(19) **Europäisches Patentamt**
**European Patent Office**
**Office européen des brevets**

(11)  **EP 1 534 122 B1**

(12)  **EUROPEAN PATENT SPECIFICATION**

(45) Date of publication and mention
of the grant of the patent:
**20.07.2016  Bulletin 2016/29**

(51) Int Cl.:
**G06F 19/20** *(2011.01)*     *G06F 19/18* *(2011.01)*
**G06F 19/24** *(2011.01)*

(21) Application number: **03788512.6**

(22) Date of filing: **15.08.2003**

(86) International application number:
**PCT/US2003/025563**

(87) International publication number:
**WO 2004/016218 (26.02.2004 Gazette 2004/09)**

(54) **MEDICAL DECISION SUPPORT SYSTEMS UTILIZING GENE EXPRESSION AND CLINICAL INFORMATION AND METHOD FOR USE**

SYSTEME ZUR UNTERSTÜTZUNG MEDIZINISCHER ENTSCHEIDUNGEN UNTER VERWENDUNG VON GENEXPRESSION UND KLINISCHEN INFORMATIONEN UND ANWENDUNGSVERFAHREN

SYSTEMES DE SUPPORT DE DECISION MEDICALE UTILISANT L'EXPRESSION GENIQUE AINSI QUE DES INFORMATIONS CLINIQUES, ET PROCEDES D'UTILISATION CORRESPONDANTS

(84) Designated Contracting States:
**AT BE BG CH CY CZ DE DK EE ES FI FR GB GR HU IE IT LI LU MC NL PT RO SE SI SK TR**

(30) Priority: **15.08.2002  US 403756 P**

(43) Date of publication of application:
**01.06.2005  Bulletin 2005/22**

(73) Proprietor: **Pacific Edge Limited**
**Dunedin (NZ)**

(72) Inventors:
• **KASABOV, Nikola, Kirilov**
**Auckland 1005 (NZ)**
• **FUTSCHIK, Matthias, Erwin**
**Dunedin (NZ)**
• **SULLIVAN, Michael, James**
**Christchurch (NZ)**
• **REEVE, Anthony, Edmund**
**Dunedin (NZ)**

(74) Representative: **Forsythe, Dominic et al**
**J A Kemp**
**14 South Square**
**Gray's Inn**
**London WC1R 5JJ (GB)**

(56) References cited:
**WO-A-01/78003      WO-A-02/47007**
**WO-A-02/059822      US-A- 6 071 236**
**US-B1- 6 317 700      US-B1- 6 385 474**
**US-B2- 6 669 631**

• **KASABOV N K ET AL: "DENFIS: dynamic evolving neural-fuzzy inference system and its application for time-series prediction" IEEE TRANSACTIONS ON FUZZY SYSTEMS IEEE USA, vol. 10, no. 2, April 2002 (2002-04), pages 144-154, XP002516617 ISSN: 1063-6706**

**Description**

**Field of the Invention**

This invention relates to diagnosis and evaluation of disease. In particular, this invention relates to systems for supporting medical decisions based on multiple sets of information relating to a patient's condition. More particularly, this invention relates to systems for supporting medical decisions based on genetic information and clinical information.

BACKGROUND

Medical diagnosis and evaluation of a patient's condition are of great concern to medical professionals. Much time is spent on obtaining information from a patient during visits to practitioners. Medical history is often a major component of a proper diagnosis. Additionally, a practitioner may request specific physiological or pathophysiological measurements be made, to facilitate understanding the patient's condition. Such clinical information has historically been a powerful tool to provide proper diagnosis and evaluation of therapy.

With the advent of increasingly widespread acquisition of genetic information from a patient, a practitioner now has an opportunity to incorporate genetic information and clinical information together, to further improve accuracy of diagnosis and evaluation of therapy. Proper diagnosis and monitoring are necessary for a practitioner to be able to make the best informed decisions about either initiating a course of therapy or for altering a therapeutic regimen to best suit a particular patient's needs.

However, there are few systems available that can be used to incorporate genetic information with a patient's clinical information to provide the practitioner with rapid, reliable information to assist in the decision making process.

In order to influence patient management in a clinical environment, medical decision support systems must have a high level of confidence. Examples of such medical systems are:

- Classification systems that classify, for example, a new tumour (a new patient's data) into existing classes of diseases;
- Prognostic systems that predict the risk of a patient for a particular disease (e.g. cardiovascular risk prognosis); and
- Prognostic systems that predict the outcome of a treatment for a particular patient and a particular drug (e.g. the outcome of a chemotherapy for a patient of DLBCL cancer, for example, see reference 1)

Many existing medical decision support systems use only one source of information, and thus the confidence of their models is not very high. For example, Shipp et al [1] described a system that uses machine learning techniques, specifically a weighted voting algorithm and support vector system, for prognostic stratification of patients based on gene expression data taken from 58 samples of 32 cured and 26 fatal cases. However their approach misclassified 23% of the patients in terms of predicting the outcome of their chemotherapy treatment. They achieved 77.6% correct prognosis of both cured and fatal cases of B-cell lymphoma cancer. The models on a similar task presented in Alizadeh et al [2] are not clinically applicable for classification purposes. Thus, there is a need for improved reliability of medical decision support systems that can overcome the shortcomings in the art.

In another aspect of the problem, there are no existing methods that help discover relationship between gene expression and clinical parameters thus making a personalised treatment of patients with different gene expression profiles according to their clinical parameters (e. g. age). It is known that some genes change their expression activity in one person over time and in different environments. Thus, there is a need for methods and systems that facilitate the discovery of related gene profiles to clinical data.

WO 02/47007 A discloses methods, devices and systems for classifying genetic conditions, diseases, tumors etc., and/or for predicting genetic diseases, and/or for associating molecular genetic parameters with clinical parameters and/or for identifying tumors by gene expression profiles etc. The disclosed methods, devices and systems have the steps of providing molecular genetic data and/or clinical data; automatic classification, prediction, association and/or identification by means of a supervising machine learning system.

Kasabov N K et al: "DENFIS: dynamic evolving neural-fuzzy inference system and its applications for time-series prediction" IEEE TRANSACTIONS ON FUZZY SYSTEMS IEEE USA, vol. 10, no. 2, April 2002, pages 144-154 discloses a type of fuzzy inference system denoted as dynamic evolving neural-fuzzy inference system (DENFIS), for adaptive online and offline learning. DENFIS evolve through incremental, hybrid (supervised/unsupervised), learning, and accommodate new input data, including new features, new classes etc., through local element tuning.

WO 02/059822 A discloses the use of Support Vector Machines (SVM) and RFE (Recursive Feature Elimination) for the identification of patterns that are useful for medical diagnosis, prognosis and treatment. SVM-RFE can be used with varied data sets.

WO 01/78003 A discloses a neural network module comprising an input layer comprising one or more input nodes arranged to receive input data, a rule base layer comprising one or more rule nodes, a layer comprising one or more output nodes, and an adaptive component arranged to aggregate selected two or more rule nodes in the rule base layer based on the input data. The invention also provides an adaptive learning system comprising one or more of the neural network modules of the disclosure. The disclosure further provides related methods of implementing a neural network module, an adaptive learning system, and a neural network computer program.

SUMMARY

The scope of the invention is defined in the appended claims.

Embodiments of this invention include novel methods for increasing the confidence of medical decision support systems by developing independent models based on (1) gene expression data, and (2) on clinical information, and combining them at a higher level. The method is extendable with the addition of other sources of information (e. g. demographic). The invention is also concerned with a novel method for the discovery of relationship between gene expression patterns and clinical parameters thus making a personalised (or a clinical group specific) treatment possible.

We have improved the prediction accuracy of a medical decision support system with the use of multiple types or classes of information about a subject's or patient's condition. In certain embodiments, gene expression information and the available clinical information are used to diagnose disease and to predict outcomes. In general, we can use different types of classifiers/predictors that relate to different sources of information or different classes of information.

Each of the classifiers may be obtaining good results for part of the overall problem, for example, for a particular class, but the combination of them provides better accuracy than any of them used individually.

BRIEF DESCRIPTION OF THE FIGURES

This invention is described with reference to specific embodiments thereof. A more complete understanding of the systems and methods can be appreciated by referring to the Figures, in which:

Figure 1 depicts a general scheme of the invention for integrating gene expression information and clinical data.
Figure 2 depicts the structural diagram of an EfuNN suitable for use with the methods of the invention.
Figure 3a depicts schematically a Venn diagram showing how two models, gene information and clinical information, can have different accuracy depending upon grouping of samples.
Figure 3b depicts a schematic diagram of how gene expression information and clinical information are correlated according to this invention in a hierarchical fashion with each other to produce a medical decision.
Figure 4 depicts relationships between different values of Beta 1 and Beta 2 in a combined model.
Figure 5 depicts a graph of accuracy of a medical decision as a function of alpha in a model of this invention.
Figure 6 depicts a segment from the structure of a multiplayer perceptron (MLP) of this invention.

DETAILED DESCRIPTION

**Medical Decision Support Systems**

A combined model for an improved decision support system includes in general of three modules, one that operates on independent microarray gene expression information, another that operates on independent clinical information, and a third that operates on integrated gene expression and clinical information for each patient. A system may contain one, two, or three of the described modules, but it can also contain more modules if more sources of information are available.

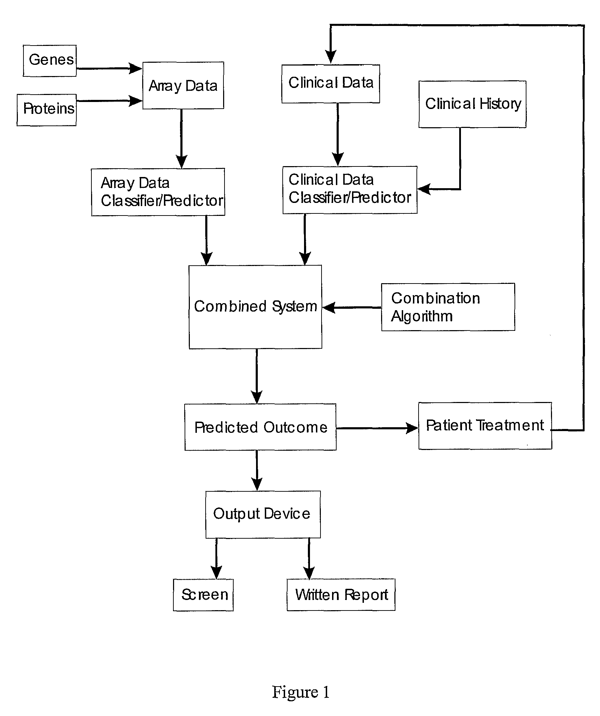

Figure 1 depicts a block diagram the flow of information in a decision support system that utilizes two sources of information, gene expression data and clinical information, for making a prognosis of the outcome of a disease and its possible treatment. The first flow of information is used in a first classifier/predictor module that is based on gene expression data. In certain embodiments the first classifier/predictor module can include EFuNN or Bayesian tools. The second flow of information is processed in a classifier/predictor module based on clinical information only. In certain embodiments, such classifier/predictor module can include an EFuNN or Bayesian tool. For a new patient, if both gene and clinical information is available, they are entered into the corresponding modules and the results are combined in a higher-level decision module using one or several models integrated at a higher level as it is described further in the invention.

This higher-level integration module combines information from two or more lower modules to produce the final prognosis for the outcome of the disease for this particular patient. Based on the suggested by the system prognosis, the best available treatment is selected.

It can be appreciated that other systems based on multiple-class analysis can be developed using the fundamental methods of this invention. The number of classifier/predictor modules can depend on the number of different classes or types of information available.

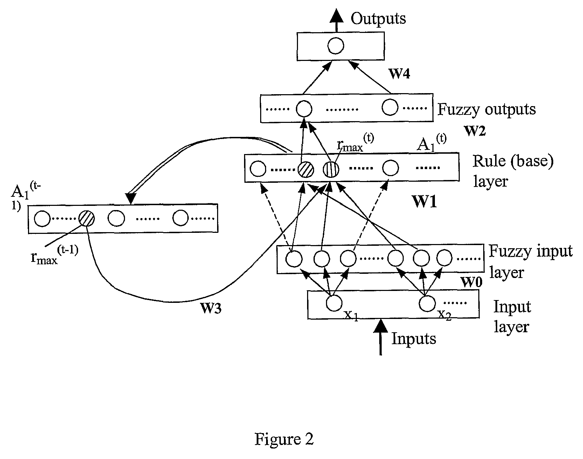

An illustration of the method is given in Figures 2-6 where two modules are used, one based on microarray gene expression data, and the other based on clinical information (in this case indicated as IPI). Figure 2 depicts an embodiment of an evolving connectionist structure (ECOS) evolving fuzzy neural network (EFuNN).

**Evolving Connectionist Systems**

In certain embodiments, the practitioner uses an adaptive learning, evolving connectionist systems (ECOS), in particular - an evolving fuzzy neural network system EFuNN, and also uses algorithms for gene expression profile (rule) extraction from ECOS [3, 4] and applies the proposed novel methodology for combining gene expression processing systems with clinical information processing systems as it is described further in the invention. The method allows for different modes of combination of gene expression and clinical data, as well as for adding new data and modules with time thus adjusting and improving the system. With the use of the proposed method the accuracy of the prognosis increases.

Using one of the modes of integration, namely an evolving connectionist system ("ECOS") trained on the integrated input vector that combines both gene expression and clinical information, rules can be extracted that relate gene expression with clinical information, so that a personalised treatment can be applied for patients in a certain group of clinical parameters (e.g. age) having a particular pattern of gene expressed in their tissue. Evolving connectionist systems are multi-modular, and can be especially useful as architectures that facilitate modelling of evolving processes and knowledge discovery. They are described further in PCT, WO 01/78003.

Briefly, an ECOS may consist of many evolving connectionist modules. An ECOS is a neural network system that operates continuously in time and adapts its structure and functionality through a continuous interaction with the environment and with other systems according to: (i) a set of parameters P that are subject to change during the system operation; (ii) an incoming continuous flow of information with unknown distribution; (iii) a goal (rationale) criteria (also subject to modification) that is applied to optimise the performance of the system over time [7].

Evolving connectionist systems can have the following specific characteristics:

(1) They can evolve in an open space, not necessarily of fixed dimensions.
(2) They can learn in on-line, pattern mode, incremental learning, fast learning - possibly by one pass of data propagation.
(3) They can learn in a life-long learning mode.
(4) They can learn as both individual systems, and evolutionary population systems.
(5) They can have evolving structures and use constructive learning.
(6) They can learn locally and locally partition the problem space, thus allowing for a fast adaptation and tracing the evolving processes over time.
(7) They can facilitate different kinds of knowledge, mostly combined memory based, statistical and symbolic knowledge.

Two distinct phases are in an ECOS operation. During a first learning phase, data vectors are fed into the system one by one with their known output values. Il a second phase (recall), a new vector is presented to the system and it calculates the output values for it.

There are different models of ECOS. One model, evolving fuzzy neural networks (EFuNN), is presented in Figure 2. This model can be used for both classification tasks and prediction tasks.

While a "neural network module" may refer to any neural network satisfying the requirements of the aspects of the invention the use of an ECOS neural network is desirably used in certain embodiments. In some of these embodiments, a neural network is exemplified in the PCT publication WO 01/78003.The algorithm describing the neural network is described further in WO 01/78003, and is set out schematically below.

**EFuNN Architecture**

EFuNNs useful for embodiments of this invention can have a five-layer structure (Figure 2). Nodes and connections are created/connected as data examples are presented. An optional short-term memory layer can be used through a feedback connection from the rule (also called, case) node layer. The layer of feedback connections could be used if temporal relationships of input data are to be memorized structurally.

The input layer represents input variables. The second layer of nodes (fuzzy input neurons, or fuzzy inputs) represents fuzzy quantification of each input variable space. For example, two fuzzy input neurons can be used to represent "small" and "large" fuzzy values. Different membership functions (MF) can be attached to these neurons (e.g., triangular, Gaussian, etc. [6, 7]. The number and the type of MF can be dynamically modified. The task of the fuzzy input nodes is to transfer the input values into membership degrees to which they belong to the corresponding MF. The layers that represent fuzzy MF are optional, as a non-fuzzy version of EFuNN can also be evolved with only three layers of neurons and two layers of connections.

The third layer contains rule (case) nodes that evolve through supervised and/or unsupervised learning. The rule nodes represent prototypes (exemplars, clusters) of input-output data associations that can be graphically represented as associations of hyper-spheres from the fuzzy input and the fuzzy output spaces. Each rule node r is defined by two vectors of connection weights - W1(r) and W2(r), the latter being adjusted through supervised learning based on the output error, and the former being adjusted through unsupervised learning based on similarity measure within a local area of the problem space. A linear activation function, or a Gaussian function, is used for the neurons of this layer.

The fourth layer of neurons represents fuzzy quantization of the output variables, similar to the input fuzzy neuron representation. Here, a weighted sum input function and a saturated linear activation function is used for the neurons to calculate the membership degrees to which the output vector associated with the presented input vector belongs to each of the output MFs. The fifth layer represents the values of the output variables. Here a linear activation function is used to calculate the defuzzified values for the output variables.

A partial case of EFuNN would be a three layer network without the fuzzy input and the fuzzy output layers. In this case a slightly modified versions of the algorithms described below are applied, mainly in terms of measuring Euclidean distance and using Gaussian activation functions.

Evolving learning in EFunNs is based on either of the following two assumptions:

(1) No rule nodes exist prior to learning and all of them are created (generated) during the evolving process; or
(2) There is an initial set of rule nodes that are not connected to the input and output nodes and become connected through the learning (evolving) process. The latter case is more biologically plausible as most of the neurons in the human brain exist before birth, and become connected through learning, but still there are areas of the brain where new neurons are created during learning if "surprisingly" different stimuli from previously seen are presented. The EFuNN evolving algorithm presented in Figure 2b does not make a difference between these two cases.

Each rule node, e.g. $r_j$, represents an association between a hyper-sphere from the fuzzy input space and a hyper-sphere from the fuzzy output space (see fig.2a), the $W1(r_j)$ connection weights representing the co-ordinates of the centre of the sphere in the fuzzy input space, and the $W2(r_j)$ - the co-ordinates in the fuzzy output space. The radius of the input hyper-sphere of a rule node $r_j$ is defined as $R_j = 1 - S_j$, where $S_j$ is the sensitivity threshold parameter defining the minimum activation of the rule node $r_j$ to a new input vector x from a new example **(x,y)** in order the example to be considered for association with this rule node.

The pair of fuzzy input-output data vectors $(x_f, y_f)$ will be allocated to the rule node $r_j$ if $x_f$ falls into the $r_j$ input receptive field (hyper-sphere), and $y_f$ falls in the $r_j$ output reactive field hyper-sphere. This is ensured through two conditions, that a local normalised fuzzy difference between $x_f$ and $W1(r_j)$ is smaller than the radius $R_j$, and the normalised output error Err= $\|y - y'\| /$ Nout is smaller than an error threshold E. Nout is the number of the outputs and y' is the produced by EFuNN output. The error parameter E sets the error tolerance of the system.

Another example of a neural network module for some aspects of the invention is an evolving classification function ("ECF"), which can be used to classify data. The learning sequence of each iteration of an ECF is described in the following steps:

1) if all vectors have been inputted, finish the current iteration; otherwise, input a vector from the data set and calculate the distances between the vector and all rule nodes already created;
2) if all distances are greater than a max-radius parameter, a new rule node is created. the position of the new rule node is the same as the current vector in the input data space and its radius is set to the min-radius parameter, and then go to step 1; otherwise:
3) if there is a rule node with a distance to the current input vector less then or equal to its radius and its class is the same as the class of the new vector, nothing will be changed and go to step 1; otherwise:
4) if there is a rule node with a distance to the input vector less then or equal to its radius and its class is different from those of the input vector, its influence field should be reduced. The radius of the new field is set to the larger value from the distance minus the min-radius, and the min-radius.
5) if there is a rule node with a distance to the input vector less then or equal to the max-radius, and its class is the same to the vector's, enlarge the influence field by taking the distance as the new radius if only such enlarged field does not cover any other rule node which has the different class; otherwise, create a new rule node the same way as in step 2, and go to step 1.

A recall (classification phase of new input vectors) in ECF is performed in the following way:

1) if the new input vector lies within the field of one or more rule nodes associated with one class, the vector belongs to this class;

2) if the input vector lies within the fields of two or more rule nodes associated with different classes, the vector will belong to the class corresponding the closest rule node.

3) if the input vector does not lie within any field, then there are two cases: (1) one-of-n mode: the vector will belong to the class corresponding the closest rule node; (2) m-of-n mode: take m highest activated by the new vector rule nodes, and calculate the average distances from the vector to the nodes with the same class; the vector will belong to the class corresponding the smallest average distance.

The above-described ECF for classification has several parameters that need to be optimized according to the data set used. These are:

1) maximum radius
2) minimum radius
3) number of membership functions (mf)
4) m-of-n value
5) number of iterations for the data presentation during learning phase.

These parameters can be optimized with the use of evolutionary computation methods, or other statistical methods, as described in [7].

An important characteristic of ECOS is that they can be used to extract rules that associate input variables (e.g. genes) to output variables (e.g. class categories). Each node in the hidden layer of the ECOS represents the center of a cluster of similar samples and can be expressed semantically as a rule. Each rule relates to the pattern of input feature levels for one or more samples belonging to a particular class from the data set. An example of what a rule might look like when extracted from the EFuNN is shown below:

IF VAR1 is LOW (0.80) and
VAR3 is HIGH (0.76) and
VAR12 is HIGH (0.91) and
VAR25 is LOW (0.80) and
VAR31 is LOW (0.87) and
THEN CLASS_Z is VERY LIKELY (with a membership degree of 0.92), accommodated Training Examples in this rule are 10 out of 50, Radius of the cluster for this rule is 0.15.

The rules are then analysed in order to identify a set of variables that are significant in distinguishing between classes. The rule extraction method described above and in reference [3] can be applied to gene expression profiling of disease as described herein and in reference [4], to find patterns of significantly expressed genes in a cluster of diseased tissues. We have unexpectedly found that ECOS described in PCT WO 01/78003 [3], and the profiling method described in PCT/480030 [4], are particularly suited for complex disease profiling based not only on gene expression information, but on a variety of information sources, including gene expression data, protein data, clinical data (for example IPI (International Prognostic Index) number (e.g., see [1]) etc. Here we combine these sources of information through ECOS to create new, more efficient prognostic and classification systems for medical applications. Using these new systems and methods enable one to discover hidden relationships between sets of genes and clinical information previously unidentifiable.

### EFuNN Learning Algorithm

To implement an EFuNN learning algorithm to the methods and systems of this invention, set initial values for the system parameters: number of membership functions; initial sensitivity thresholds (default Sj=0.9); error threshold E; aggregation parameter Nagg - number of consecutive examples after each aggregation is performed; pruning parameters OLD an Pr; a value for m (in m-of-n mode); maximum radius limit Rmax; thresholds $T_1$ and $T_2$ for rule extraction.

Set the first rule node $r_0$ to memorise the first example (x,y):

$$W1(r_0)=x_f, \text{ and } W2(r_0)=y_f;$$

*Loop* over presentations of new input-output pairs (x,y)

```
{
  Evaluate the local normalised fuzzy distance D between xf and the
  existing rule node connections W1 (formulae (1))
  Calculate the activation A1 of the rule node layer. Find the closest rule
  node rk (or the closest m rule nodes in case of m-of-n mode) to the fuzzy
  input vector xf for which A1 (rk) >= Sk (sensitivity threshold for the node
  rk),
   if there is no such a node, create a new rule node for (xf, yf)
     else
             Find the activation of the fuzzy output layer A2=W2.A1(1-
             D(W1,xf))) and the normalised output error Err= ‖y- y'‖/ Nout.
             if Err > E
               create a new rule node to accommodate the current example
               (xf, yf)
             else
                     Update W1 (rk) and W2(rk) according to (2) and (3) (in
                     case of m-of-n system update all the m rule nodes with
                     the highest A1 activation).
  Apply aggregation procedure of rule nodes after each group of Nagg
  examples are presented.
  Update the values for the rule node rk parameters Sk, Rk, Age(rk), TA (rk).
  Prune rule nodes if necessary, as defined by pruning parameters.
  Extract rules from the rule nodes (
          }
```

In certain embodiments, EFuNN techniques have certain advantages when compared with the traditional statistical and neural network techniques, including: (i) they can have a flexible structure that reflects the complexity of the data used for their training; (ii) they can perform both clustering and classification/prediction; (iii) models can be adapted on new data without the need to be retrained on old data; (iv) they can be used to extract rules (profiles) of different sub-classes of samples.

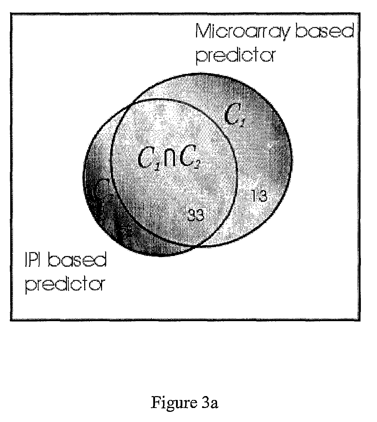

Figure 3a illustrates the method from Figure 1 in the notation of the set theory, where two modules C1 and C2 are used to classify two-class data (fatal and cured as described in [1]). Two models, in this case, one based on microarray gene expression data and the other on clinical information (in this case indicated as IPI), may have different accuracy depending on the grouping of the data samples. For example, 13 samples are predicted correctly only by module 1, 8 samples are predicted correctly by only module 2, and 33 samples are predicted correctly by the two modules. In this case, only two modules and two classes are used, but it can be appreciated that any number of modules and any number of classes can be used. The correctly predicted 54 samples out of 58 used in this case sets a boundary of 93% possible classification that could be eventually achieved with the use of this model.

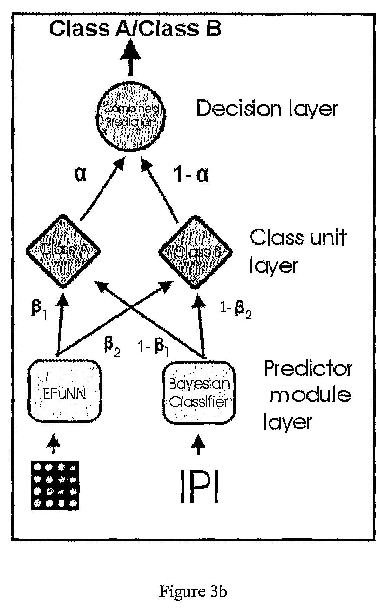

Figure 3b depicts a block diagram of the method from Figure 1, applied on a two-class problem (class and A and class B as is the case described in [1]) is depicted in Figure 3a. Two modules C1 and C2 from Figure 3a for the classification of the two classes A and B are combined in Figure 3b in a hierarchical manner.

Figure 3b depicts a three-layered system of this invention. The two modules from Figure 1 are combined in a hierarchical manner. For example, the first module that operates on microarray gene expression data uses EFuNN, and the second module that operates on clinical (IPI in the case) information is a Bayesian classifier. In principle, for each of these modules any type of classifier/predictor can be used (e.g. neural networks, support vector machines, rule-based systems, decision trees, statistical methods and the like), and in some embodiments, an ECOS of the EFuNN or ECF type as described in this invention above.

It can be appreciated that additional layers can be applied depending on the numbers of modules and the types of classifiers or predictors available to be used. For example, in other embodiments, neural networks, support vector machines, rule-based systems, decision trees, and statistical methods can be used to construct a more complex medical decision support system. Thus, it can be appreciated that medical decision support systems can have more than three layers.

A first layer constitutes the modules themselves, each of them trained and tested with parameters optimised on the different data sets available (the different sources of information). It may be desirable to try different classification/prediction models for each of the modules and then chose the best one in terms of smallest residual error. Error minimization methods are well known in the art and need not be described further herein.

A second layer constitutes classes. All the class-elements of the second layer are fully connected with the module-elements of the first layer. A third layer in this example is the final outcome element, a combined output from all class-elements of the second layer. The third layer element is connected fully to the previous layer elements.

Elements from the different layers are connected through connection weights $\beta 1$, $1- \beta 1$, $\beta 2$, $1- \beta 2$, and $\alpha$ as

shown in Figure 3b. To evaluate and quantify parameter values $\beta1$, $\beta2$, and $\alpha$ for the combined decision support system from Figure 3b, at least three methods can be used.

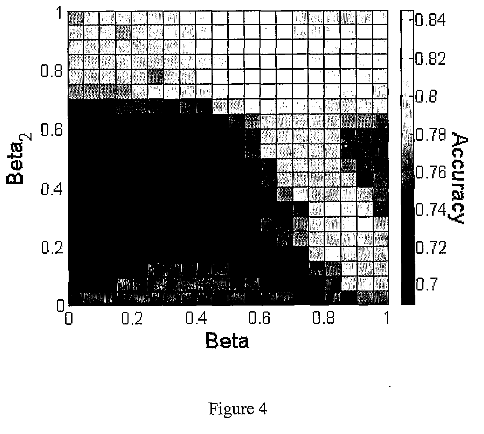

(1) The first method is based on an exhaustive search in the parameter values space, so for every combination of the parameter values a new system is generated and tested. The parameter values that give the system the highest accuracy are selected for the use in the medical decision support system in a clinical environment. Figure 4 shows the accuracy of the combined system for different values of $\beta1$ and $\beta2$. We have found that the best accuracy is achieved for certain interval values for $\beta1$ and $\beta2$. The selected values are $\beta1= \beta2=0.75$.

Figure 4 depicts results of such an exhaustive search method. Different values of Beta1 and Beta2 in the combined model (see Figure 2) give different accuracy of prediction. The optimal values can be found through exhaustive search. During the exhaustive search procedure, all values for $\alpha$ are tested as shown in Figure 4. The figure shows the process of testing the exhaustive search method for finding an optimum value for $\alpha$ for the example model from Figure 3. A value of 0.4 was found to produce desirably improved results compared to prior methods.

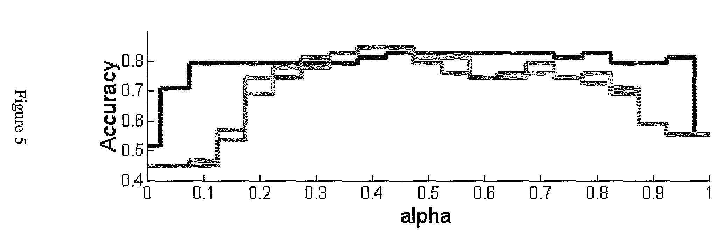

Figure 5 depicts the results applying the exhaustive search method for finding an optimum value for $\alpha$ for the example model from Figure 2 using the following method.

Step 1. Create 2 modules, one for gene expression data and one for clinical information data (in case of only two modules used) through training, testing, and parameter optimisation.

Step 2. For every value of $\beta1$ & for every value of $\beta2$ & for every value of $\alpha$, DO

Test the accuracy of the combined system on the whole data set.

Step 3. Chose the parameter values $\beta1$, $\beta2$, and $\alpha$ that give the highest accuracy of the combined system.

## Example of the First Approach

For the B-Lymphoma case study [1], values for the IPI (International Prognostic Index) are available for each of the 58 samples. Samples were stratified into 5 strata according to their IPI as follows:

Stratum 1: IPI = low: 26 samples, of which 19 belong to the *cured* class and 7 to the *fatal* class.
Stratum 2: IPI = low intermediate: 11 samples, of which 7 belong to the *cured* class and 4 to the *fatal* class.
Stratum 3: IPI = high intermediate: 17 samples, of which 4 belong to the *cured* class and 13 to the *fatal* class.
Stratum 4: IPI = high: 2 samples, of which 2 belong to the *fatal* class. Stratum 5: IPI = unknown: 2 samples. These samples were assigned the same IPI number as the closest samples based on the gene expression data and on the Euclidean distance.

Using the IPI stratification, a single prediction factor and a Bayesian classifier gives a prediction accuracy of 73.2%. Using gene expression data and an EFuNN classifier gives an accuracy of 78.5%. The combined model accuracy, for $\beta1= \beta2=0.75$ and $\alpha=0.4$, gives an overall accuracy of 87.5% if 56 examples are used for the first module. If the number of the IPI examples increases the overall accuracy of the first module increases because the IPI module is independent from the gene expression module (see reference [1]).

(2) The second method is a statistically based specialization method used for the selection of optimal parameter values. Each class output of each module is weighted with the normalised class accuracy calculated for this module across all the modules in the system. Continuous output values for the class outputs (e.g. 0.8 rather than 1) are multiplied by the weights, and the sum of the weighted output values from all modules constitutes the final output value for the class. A class is chosen with the highest output value. This is similar to the principle of statistically based specialisation [5]. The method is illustrated on the following example.

## Example of the Second Approach

Step 1: Assume that the combined model consists of three modules:

1) A gene expression data module (e.g. N genes as inputs and 2 classes as outputs, class A and class B);
2) A clinical information module (e.g. M inputs, binary encoding of the clinical information, and 2 classes as outputs); and
3) An integrated information module (e.g. N+M inputs: the N gene expression variables plus the M clinical input variables and 2 class outputs)

Step 2: Assume that each of the three modules produces different prognostic accuracy as follows: module one:

90% (88 and 92 for each class); module two: 70% (65 and 75 for each class respectively) and module 3: 80% (75% and 85% for each class respectively). By using a combined output at a higher-level decision module for the final prognostic evaluation, the total accuracy increases to 92% as explained below.

Having quantified the accuracy of each of the three modules for each of the two classes, the following connection weights are calculated for each of the three modules for class A:

Module 1: 0.88/2.28 =0.39
Module 2: 0.65/2.28= 0.28
Module 3: 0.75/2.28 = 0.33

The following weights are calculated for each of the three modules for class B:

Module 1: 0.92/2.62=0.35;
Module 2: 0.85/2.62=0.325
Module 3: 0.85/2.62 =0.325;

Based on the above coefficients, one calculates the accuracy of the classification of samples for each of the classes A and B, for example, 0.8 and 0.9.

The final decision in the third layer output element can be taken either as the maximum value between the calculated class-output for each class elements or by applying a calculated third layer coefficients for combining the class outputs as follows:

Class A - to third layer output coefficient: 0.8 / (0.8 +0.9) = 0.47;
Class B - to third layer output coefficient: 0.9 / (0.8 +0.9) = 0.53;

If for a new input vector the corresponding output values for each class of each of the three modules are respectively: 0.6 and 0.5; 0.4 and 0.6; 0.5 and 0.5, the output value for class A is calculated as:

$$(0.6 \times 0.39) + (0.4 \times 0.28) + (0.5 \times 0.33) \simeq 0.51.$$

The output value for class B is calculated as:

$$(0.5 \times 0.35) + (0.6 \times 0.325) + (0.5 \times 0.325) = 0.53.$$

The predicted outcome according to the maximizing strategy is class B.

According to the weighed output strategy, the output will be B again as output class A will get final evaluation of 0.51 x 0.47=0.24, and output class B will get an evaluation of: 0.53 x 0.53=0.28.

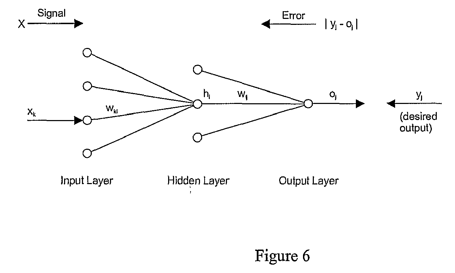

(3) In a third method, a combined system is interpreted as a multi-layer perceptron as described below and shown in Figure 6. Optimal parameter values are calculated through a learning procedure utilising the error back-propagation algorithm (see for example the algorithm from p.276 [6]) also shown in Figure 7.

Parameter values attached to the connections in a multi-layer perceptron (MLP) neural network structure (see the example shown in Figure 3b) are calculated as connection weights during training of the neural network.

The invention is also concerned with the discovery of patterns of gene expression in clusters of tissues (groups of samples that have a similar gene expression profiles) related to particular pattern of clinical parameters (e.g. low IPI, old age and low blood pressure). This is an important discovery because different clinical groups may have different patterns of gene expression that makes the process of finding common genes and drug targets for the whole population impossible. The method is described below:

**Methods for Discovering Patterns of Genes Related to a Group of Patients Characterized by a Particular Clinical Parameter**

One can also use an ECOS module with the combined input vector (N gene expression variables and M clinical variables) to derive patterns of gene expression as they relate to clinical findings. After a module is trained on data as described above, rules that represent clusters of data in the input space (which is the combined gene expression and clinical variables space) can be extracted as described above. As applied to a combined inputs module, these rules will have both gene variables and clinical variables that define the cluster of data samples expressed by this rule, thus uncovering the relationship between the genes and the clinical parameters as captured in the rule. For example:

IF VAR1 =Gene1 is LOW (0.80) and
VAR3=Gene15 is HIGH (0.76) and
VAR12= Gene 32 is HIGH (0.91) and
VAR25= Age is LOW (0.80) and
VAR31= IPI is LOW (0.87)
THEN CLASS Survive is VERY LIKELY (with a membership degree of 0.9), accommodated training examples in this rule are 7 out of 26. Radius of the cluster for this rule is 0.2.

The above rule is interpreted as follows: for young people with low IPI, if gene 1 is low expressed and genes 15 and 32 are highly expressed, than the chances of the person to survive after the treatment are very high, measured as 90%.

Figure 6 depicts a segment from the structure of a multiplayer perceptron (MLP). Using the structure shown in Figure 6, one can implement an algorithm as follows:

Forward pass:

BF1.    Apply an input vector x and its corresponding output vectory (the desired output).

BF2.    Propagate forward the input signals through all the neurons in all the layers and calculate the output signals.

BF3.    Calculate the $Err_j$ for every outputneuronj as for example: $Err_j = y_j - o_j$, where $y_j$ is the jth element of the desired output vector y.

Backward pass:

BB1.    Adjust the weights between the intermediate neurons i and output neurons j according to the calculated error: $\Delta w_{ij}(t+1)= = 1rate. o_j (1-o_j). Err_j. o_i, + momentum. \Delta w_{ij} (t)$
BB2.    Calculate the error $Err_i$ for neurons i in the intermediate layer: $Err_i = \Sigma Err_j. w_{ij}$
BB3.    Propagate the error back to the neurons k of lower level: $\Delta w_{ki}(t+1)=1rate.o_i(1-o_i). Err_i.x_k + momentum. \Delta w_{ki}(t)$

The descriptions and examples herein are intended to illustrate embodiments of the invention, and are not intended to be limiting to the scope of the invention. Other embodiments based on the descriptions, examples and Figures can be produced and practiced by those of ordinary skill in the art, without departing from the scope of the present invention as defined by the claims.

References

[1] M. Shipp, et al, Diffuse large B-cell lymphoma outcome prediction by gene-expression profiling and supervised machine learning, Nature Medicine, vol.8, n.1, January 2002, 68-74
[2] Alizadeh et al, Distinct types of diffuse large B-cell lymphoma identified by gene-expression profiling, Nature, vol.403, February 2000, 503-511
[3] N. Kasabov, Adaptive system and method, PCT WO 01/78003, 2001
[4] A.Reeve, M. Futschik, M.Sullivan, N. Kasabov, and P. Guildford, Medical applications of Adaptive Learning Systems Using Gene Expression Data, PCT/480030, 7/03/2003, priority date 15/03/2002
[5] N. Kasabov, E. Postma, and J. van den Herik, AVIS: a connectionist-based framework for integrated auditory and visual information processing, Information Sciences, vol. 123, 127-148 (2000)
[6] N. Kasabov, Foundations of neural networks, fuzzy systems, and knowledge engineering, MIT Press, 1996
[7] N.Kasabov, Evolving connectionist systems: methods and applications in bioinformatics, brain study and intelligent machines, Springer Verlag, 2002

**Claims**

1. A computer-implemented method for supporting a medical decision, **characterized by** comprising:

   providing a three layer system comprising:

   a. a first layer comprising:

   i. a first predictor module-element that operates on microarray gene expression data using an evolving fuzzy neural network;
   ii. a second predictor module-element that operates on clinical information using a Bayesian classifier;

   b. a second layer comprising the following class-elements:

   i. Class A; and
   ii. Class B; and

   c. a third layer consisting of an output element for providing a combined output by combining the output from all class-elements of the second layer, wherein:

   said third layer output element is connected fully to the class-elements of the second layer through connection weights $\alpha$ and 1- $\alpha$;
   all the class-elements of the second layer are fully connected with the module-elements of the first layer through connection weights $\beta_1$, 1- $\beta_1$, $\beta_2$ and 1-$\beta_2$; and
   said connection weights are such as to minimize an error of said combined output such that said combined output has a greater accuracy than either Class A output or Class B output individually, wherein:

   the parameter values $\beta_1$, $\beta_2$, and $\alpha$ are evaluated and quantified for the three layer system using an exhaustive search method according to the steps:

   i. create said first predictor module-element and said second predictor module-element through training, testing, and parameter optimisation;
   ii. for every value of $\beta_1$ and for every value of $\beta_2$ and for every value of $\alpha$, test the accuracy of the three layer system for the whole of a data set; and
   iii. choose the parameter values $\beta_1$, $\beta_2$, and $\alpha$ that give the highest accuracy of the three layer system;

   the parameter values $\beta_1$, $\beta_2$, and $\alpha$ are evaluated and quantified for the three layer system according to a statistically based specialization method, where each class output of each module is weighted with the normalized class accuracy calculated for this module across all the modules in the system, wherein continuous output values for the class outputs are multiplied by the weights, and the sum of the weighted output values from all modules constitutes the final output value for the class, and wherein a class is chosen with the highest output value; or
   the three layer system is interpreted as a multi-layer perceptron, where the parameter values $\beta_1$, $\beta_2$, and $\alpha$ are evaluated and quantified for the three layer system through a learning procedure utilising the error back-propagation algorithm, wherein parameter values attached to the connections in a multi-layer perceptron neural network structure are calculated as connection weights during training of the neural network.

2. The method of claim 1, in the case where the three layer system is interpreted as a multi-layer perceptron, wherein said algorithm is implemented as follows:

   forward pass:

   BF1: apply an input vector x and its corresponding output vector y (the desired output),
   BF2: propagate forward the input signals through all the neurons in all the layers and calculate the output signals,

BF3: calculate the $Err_J$ for every output neuron j as $Err_J = y_j - o_j$, where $y_j$ is the $j^{th}$ element of the desired output vector y; and

backward pass:

BB1: adjust the weights between the intermediate neurons i and output neurons j according to the calculated error:

$$\Delta w_{ij}\,(t+1) = Irate.o_j\,(1-o_j).\,Err_j.\;o_i + momentum.\,\Delta w_{ij}\,(t)\;;$$

BB2 : calculate the error $Err_j$ for neurons i in the intermediate layer :

$$Err_i = \sum Err_j.w_{ij};$$

BB3: propagate the error back to the neurons k of lower level:

$$\Delta w_{ki}\,(t+1) = Irate.o_j\,(1-o_j).\,Err_j.\,x_k + momentum.\;\Delta w_{ki}\,(t).$$

3. The method of claim 1, in the case where the parameter values are evaluated and quantified using the exhaustive search method, wherein $\beta_1 = \beta_2 = 0.75$, and $\alpha = 0.4$.

4. A medical decision support system for performing the method according to claim 1, 2 or 3, comprising:

> a processor;
> a memory device;
> a first input for acquiring said microarray gene expression data, said first input being associated with said first predictor module-element;
> a second input for acquiring said clinical information, said second input being associated with said second predictor module-element; and
> a program available to said processor for causing said processor to carry out the method according to claim 1, 2 or 3.

**Patentansprüche**

1. Computer-implementiertes Verfahren zur Unterstützung einer medizinischen Entscheidung, **dadurch gekennzeichnet, dass** es umfasst:

Bereitstellen eines Dreischichtensystems, umfassend:

> a. eine erste Schicht, umfassend:

>> i. ein erstes Prädiktormodulelement, das mit Mikroarray-Genexpressionsdaten unter Verwendung eines Entwicklungs-Fuzzy-Neuralnetzwerks arbeitet;
>> ii. ein zweites Prädiktormodulelement, das mit klinischen Informationen unter Verwendung eines Bayesian-Klassifizierers arbeitet;

> b. eine zweite Schicht, die die folgenden Klasse-Elemente umfasst:

>> i. Klasse A und
>> ii. Klasse B, und

> c. eine dritte Schicht, bestehend aus einem Output-Element zur Bereitstellung eines kombinierten Outputs durch Kombinieren des Outputs von allen Klasse-Elementen der zweiten Schicht, wobei:

das dritte Schicht-Output-Element vollständig mit den Klasse-Elementen der zweiten Schicht durch Verbindungsgewichte $\alpha$ und $1 - \alpha$ verbunden ist;

alle Klasse-Elemente der zweiten Schicht vollständig mit den Modulelementen der ersten Schicht durch Verbindungsgewichte $\beta 1$, $1-\beta_1$, $\beta_2$ und $1-\beta_2$ verbunden sind und

die genannten Verbindungsgewichte so sind, dass sie einen Fehler des kombinierten Outputs minimieren, so dass der kombinierte Output eine größere Genauigkeit als Klasse-A-Output oder Klasse-B-Output einzeln hat, wobei:

die Parameterwerte $\beta_1$, $\beta_2$ und $\alpha$ für das Dreischichtensystem unter Verwendung eines umfassenden Suchverfahrens gemäß den folgenden Schritten evaluiert und quantifiziert werden:

i. Schaffen des ersten Prädiktormodulelements und des zweiten Prädiktormodulelements durch Training, Untersuchung und Parameteroptimierung;
ii. für jeden Wert von $\beta_1$ und für jeden Wert von $\beta_2$ und für jeden Wert von $\alpha$ Untersuchen der Testgenauigkeit des Dreischichtensystems für die Gesamtheit eines Datensatzes und
iii. Auswählen der Parameterwerte $\beta_1$, $\beta_2$ und $\alpha$, die die höchste Genauigkeit des Dreischichtensystems ergeben;

wobei die Parameterwerte $\beta_1$, $\beta_2$ und $\alpha$ für das Dreischichtensystem gemäß einem statistisch basierten Spezialisierungsverfahren evaluiert und quantifiziert werden, wobei jeder Klasse-Output jedes Moduls mit der normalisierten Klasse-Genauigkeit, die für dieses Modul errechnet wurde, durch alle Module im System gewichtet wird, wobei kontinuierliche Output-Werte für die Klasse-Outputs mit den Gewichten multipliziert werden und die Summe der gewichteten Output-Werte von allen Modulen den End-Output-Wert für die Klasse bildet und wobei eine Klasse mit dem höchsten Output-Wert ausgewählt wird, oder

das Dreischichtensystem als ein Mehrschichten-Perzeptron interpretiert wird, wobei die Parameterwerte $\beta_1$, $\beta_2$ und $\alpha$ für das Dreischichtensystem durch einen Lernvorgang, der den Fehler-Back-Propagations-Algorithmus nutzt, evaluiert und quantifiziert werden, wobei die Parameterwerte, die mit den Verbindungen in einer Mehrschichten-Perzeptron-Neuralnetzwerkstruktur verknüpft sind, während eines Trainings des Neuralnetzwerkes als Verbindungsgewichte errechnet werden.

**2.** Verfahren gemäß Anspruch 1, wobei in dem Fall, in dem das Dreischichtensystem als ein Mehrschichten-Perzeptron interpretiert wird, der Algorithmus wie folgt implementiert wird:

Vorwärtspass:

BF1: Anwenden eines Input-Vektors x und seines entsprechenden Output-Vektors y (der gewünschte Output),
BF2: Fortschreiten der Inputsignale nach vorne durch alle Neuronen in allen Schichten und Errechnen der Output-Signale,
BF3: Errechnen des $Err_J$ für jedes Output-Neuron j als $Err_J = y_j - o_j$, worin $y_j$ das j-te Element des gewünschten Output-Vektors y ist, und

Rückwärtspass:

BB1: Einstellen der Gewicht zwischen den Zwischenneuronen i und den Output-Neuronen j entsprechend dem errechneten Fehler:

$$\Delta w_{ij} \, (t+1) = Irate.o_j \, (1-o_j). \, Err_j. \, o_i + momentum. \, \Delta w_{ij} \, (t);$$

BB2: Errechnen des Fehlers $Err_j$ für Neuronen i in der Zwischenschicht:

$$Err_i = \Sigma Err_j.w_{ij};$$

BB3: Fortschreiten des Fehlers zurück zu den Neuronen k vom niedrigeren Level:

$$\Delta w_{ki}\ (t+1) = \text{Irate}.o_j\ (1-o_j).\ \text{Err}_j.\ x_k + \text{momentum}.\ \Delta w_{ki}\ (t).$$

**3.** Verfahren gemäß Anspruch 1, wobei im Fall, in dem die Parameterwerte unter Verwendung des umfassenden Suchverfahrens evaluiert und quantifiziert werden, $\beta_1 = \beta_2 = 0{,}75$ und $\alpha = 0{,}4$.

**4.** System zur Unterstützung einer medizinischen Entscheidung zur Durchführung des Verfahrens gemäß Anspruch 1, 2 oder 3, umfassend:

> einen Prozessor;
> eine Speichereinheit;
> einen ersten Input zur Erfassung der Mikroarray-Genexpressionsdaten, wobei der erste Input mit dem ersten Prädiktormodulelement assoziiert ist;
> einen zweiten Input zur Erfassung der klinischen Informationen, wobei der zweite Input mit dem zweiten Prädiktormodulelement assoziiert ist, und
> ein Programm, das für den Prozessor verfügbar ist, zur Bewirkung, dass der Prozessor das Verfahren gemäß Anspruch 1, 2 oder 3 ausführt.

**Revendications**

**1.** Procédé mis en oeuvre par ordinateur pour servir d'aide à la décision médicale, **caractérisé en ce qu'**il comprend :

la mise en place d'un système à trois couches, comprenant :

> a. une première couche, comportant :
>
> > i. un premier élément-module prédicteur qui fonctionne sur la base de données d'expression génétique en micro-réseau en utilisant un réseau neuro-flou évolutif;
> > ii. un deuxième élément-module prédicteur qui fonctionne sur la base d'informations cliniques en utilisant un classifieur bayésien;
>
> b. une deuxième couche comportant les éléments de classe suivants :
>
> > i. Classe A; et
> > ii. Classe B; et
>
> c. une troisième couche constituée d'un élément de sortie pour fournir des données de sortie combinées en combinant les données de sortie de tous les éléments de classe de la deuxième couche, où

ledit élément de sortie de troisième couche est connecté intégralement aux éléments de classe de la deuxième couche, par l'intermédiaire de poids de connexion $\alpha$ et $1-\alpha$ ;
tous les éléments de classe de la deuxième couche sont connectés intégralement aux éléments-modules de la première couche, par l'intermédiaire de poids de connexion $\beta_1$, $1-\beta_1$, $\beta_2$ et $1-\beta_2$ ; et
lesdits poids de connexion sont tels qu'ils réduisent à un minimum une erreur desdites données de sortie combinées, de manière à ce que lesdites données de sortie combinées présentent une exactitude supérieure aussi bien à celle des données de sortie de Classe A que des données de sortie de Classe B, individuellement, où :

> les valeurs de paramètres $\beta_1$, $\beta_2$ et $\alpha$ sont évaluées et quantifiées pour le système à trois couches en utilisant un procédé de recherche exhaustive, conformément aux étapes suivantes :
>
> > i. création dudit premier élément-module prédicteur et dudit deuxième élément-module prédicteur, par apprentissage, essai et optimisation de paramètres;
> > ii. pour chaque valeur de $\beta_1$ et pour chaque valeur de $\beta_2$ et pour chaque valeur de $\alpha$, essai du système à trois couches pour l'ensemble d'un jeu de données; et
> > iii. choix des valeurs de paramètres $\beta_1$, $\beta_2$ et $\alpha$, qui offrent la plus grande exactitude du système à trois couches;

les valeurs de paramètres $\beta_1$, $\beta_2$ et $\alpha$ sont évaluées et quantifiées pour le système à trois couches, conformément à un procédé de spécialisation s'appuyant sur des statistiques, où toutes les données de sortie de classe de chaque module sont pondérées avec l'exactitude de classe normalisée calculée pour ce module à travers tous les modules dans le système, où des valeurs de sortie continues pour les données de sortie de classe sont multipliées par les coefficients de pondération, et la somme des valeurs de sortie pondérées de tous les modules constitue la valeur de sortie finale pour la classe, et où une classe est choisie avec la valeur de sortie la plus élevée; ou

le système à trois couches est interprété en tant que perceptron multicouche, où les valeurs de paramètres $\beta_1$, $\beta_2$ et $\alpha$ sont évaluées et quantifiées pour le système à trois couches, à travers une procédure d'apprentissage utilisant l'algorithme de rétropropagation de l'erreur, où les valeurs de paramètres attachées à la connexion dans une structure de réseau neuronal à perceptron multicouche sont calculées en tant que poids de connexion pendant l'apprentissage du réseau neuronal.

2. Procédé selon la revendication 1, dans le cas où le système à trois couches est interprété en tant que perceptron multicouche, où ledit algorithme est mis en oeuvre de la manière suivante :

Passage avant :

BF1 : appliquer un vecteur d'entrée x et son vecteur de sortie y correspondant (la sortie souhaitée),
BF2 : propager vers l'avant les signaux d'entrée à travers tous les neurones dans toutes les couches et calculer les signaux de sortie,
BF3 : calculer $Err_J$ pour chaque neurone de sortie j, en tant que $Err_J = yj - o_j$, où $y_j$ est le $j^{ème}$ élément du vecteur de sortie y souhaité; et

Passage arrière :

BB1 : régler les poids entre les neurones intermédiaires i et les neurones de sortie j, en fonction de l'erreur calculée :

$$\Delta w_{ij}(t+1) = Irate.o_j(1\text{-}o_j). Err_j.o_j + momentum, \Delta w_{ij}(t);$$

BB2 : calculer l'erreur Errj pour les neurones i dans la couche intermédiaire :

$$Err_i = \Sigma Err_j.w_{ij};$$

BB3 : propager l'erreur en arrière vers les neurones k du niveau inférieur :

$$\Delta w_{ki}(t+1) = Irate.o_j(1\text{-}o_j). Err_j.x_k + momentum. \Delta w_{ki}(t)$$

3. Procédé selon la revendication 1, dans le cas où les valeurs de paramètres sont évaluées et quantifiées en utilisant la méthode de recherche exhaustive, où $\beta_1 = \beta_2 = 0,75$, et $\alpha = 0,4$.

4. Système d'aide à la décision médicale destiné à mettre en oeuvre le procédé selon la revendication 1, 2 ou 3, comprenant :

un processeur;
un dispositif de mémoire;
une première entrée destinée à acquérir des données d'expression génétique en micro-réseau, ladite première entrée étant associée audit premier élément-module prédicteur;
une deuxième entrée destinée à acquérir lesdites informations cliniques, ladite deuxième entrée étant associée audit deuxième élément-module prédicteur; et
un programme disponible pour ledit processeur en vue de faire en sorte que ledit processeur exécute le procédé selon la revendication 1, 2 ou 3.

Figure 1

Figure 2

Figure 3a

Figure 3b

Figure 4

Figure 5

22

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0247007 A
- WO 02059822 A
- WO 0178003 A
- WO 480030 A

### Non-patent literature cited in the description

- **KASABOV N K et al.** DENFIS: dynamic evolving neural-fuzzy inference system and its applications for time-series prediction. *IEEE TRANSACTIONS ON FUZZY SYSTEMS IEEE USA,* April 2002, vol. 10 (2), 144-154
- **M. SHIPP et al.** Diffuse large B-cell lymphoma outcome prediction by gene-expression profiling and supervised machine learning. *Nature Medicine,* January 2002, vol. 8 (1), 68-74
- **ALIZADEH et al.** Distinct types of diffuse large B-cell lymphoma identified by gene-expression profiling. *Nature,* February 2000, vol. 403, 503-511
- **N. KASABOV ; E. POSTMA ; J. VAN DEN HERIK.** AVIS: a connectionist-based framework for integrated auditory and visual information processing. *Information Sciences,* 2000, vol. 123, 127-148
- **N. KASABOV.** Foundations of neural networks, fuzzy systems, and knowledge engineering. MIT Press, 1996
- **N.KASABOV.** Evolving connectionist systems: methods and applications in bioinformatics, brain study and intelligent machines. Springer Verlag, 2002